# EUROPEAN PATENT SPECIFICATION

(11) **EP 3 593 774 B1**
(45) Date of publication and mention of the grant of the patent: **01.06.2022**
(21) Application number: 18787519.0
(22) Date of filing: 13.04.2018
(51) Int. Cl.: A61F 13/00, A61F 13/10, B32B 5/24, B32B 7/14

(54) **COVERING ARTICLE**
ABDECKARTIKEL
ARTICLE DE COUVERTURE

(30) Priority: 19.04.2017 US 201762487256 P
(43) Date of publication of application: 15.01.2020
(73) Proprietor: Yamada, Kikuo, Shinagawa-ku Tokyo 141-0022 (JP)
(72) Inventor: Yamada, Kikuo, Shinagawa-ku Tokyo 141-0022 (JP)
(74) Representative: Algemeen Octrooi- en Merkenbureau B.V.
(86) International application number: PCT/JP2018/015541
(87) International publication number: WO 2018/193987

(56) References cited:
- WO-A1-84/03832
- WO-A1-2016/093371
- WO-A1-2016/181576
- JP-A- 2002 516 151
- JP-U- 3 018 487
- JP-U- S5 533 785
- JP-U- S59 143 419
- US-A- 6 071 834
- US-A1- 2003 040 691

## Description

### Technical Field

The present invention relates to a covering article that covers a part of a body and is stretchable and waterproof.

### Background Art

A covering article is generally used that directly covers an affected part of a body to protect the affected part, or holds a gauze coated with a chemical agent to be applied to the affected part. It has been inconvenient because a user has to remove the covering article or devise so as not to wet the covering article when the user performs hand washing, cooking, washing, and bathing.

On the other hand, for example, Patent Literature 1 discloses a covering article in which a fabric to hold a chemical agent and a waterproof film are laminated. According to this covering article, it is possible to prevent permeation of water and leakage of a chemical agent in performing household work such as washing with water.

### Citation List

### Patent Literature

Patent Literature 1: JP 2017-131400 A

US2003/0040691 A1 discloses an elastic bandage having a nonelastic absorbent nonwoven web, a non-elastic breathable nonwoven web, and a meltspun elastomeric material disposed between the two webs.

The meltspun elastomeric material can comprise a plurality of meltspun elastomeric filaments aligned in substantially parallel distribution in a machine direction, the filaments attached to a side of the nonelastic breathable nonwoven web and to a side of the nonelastic absorbent nonwoven web. The nonelastic absorbent nonwoven web can comprise a laminate of nonwoven layers. The non-elastic breathable nonwoven web can comprise a breathable film bonded to a nonwoven layer or laminate individual nonwoven layers. A self adherent coating may be disposed upon an outer surface of the elastic bandage.

### Summary of Invention

### Technical Problem

However, the covering article disclosed in Patent Literature 1 described above uses a stretchable fabric and a stretchable film, which causes high cost and requires washing of the covering article in order to hygienically use for a long period of time. During the washing, it has been necessary to wear another covering article or to refrain from wearing itself, which puts a burden on the user.

An object of the present invention is to provide a covering article that has a simple configuration and reduces the cost.

### Solution to Problem

A covering article according to the present invention is a covering article for a body part according to claim 1.

### Advantageous Effects of Invention

According to the present invention, it is possible to achieve cost reduction with a simple configuration. Brief Description of Drawings

Fig. 1 is a plan view of a stretchable waterproof sheet according to an embodiment of the present invention.
Fig. 2 is a cross-sectional view taken along line A-A of Fig. 1.
Fig. 3 is a view showing a covering article using the stretchable waterproof sheet shown in Fig. 1.
Fig. 4 is a view showing a state where the covering article shown in Fig. 3 is worn on an elbow.
Fig. 5 is a view showing a state where the covering article shown in Fig. 3 is worn on a finger.
Fig. 6 is view showing a variation of a layer configuration of the stretchable waterproof sheet.

### Description of Embodiments

Hereinafter, an embodiment of the present invention will be described with reference to the drawings.

### One Embodiment

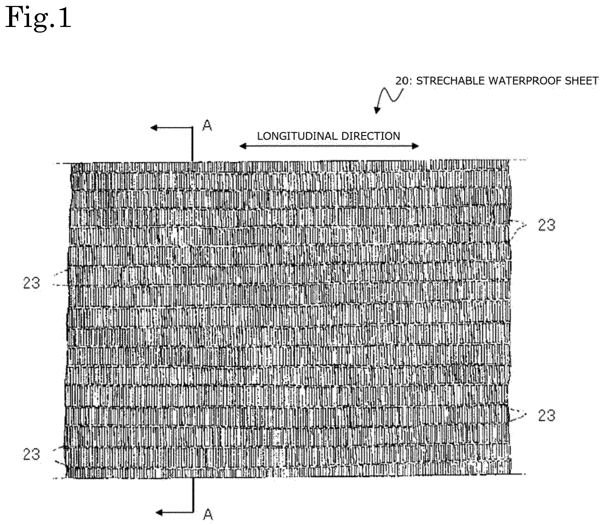

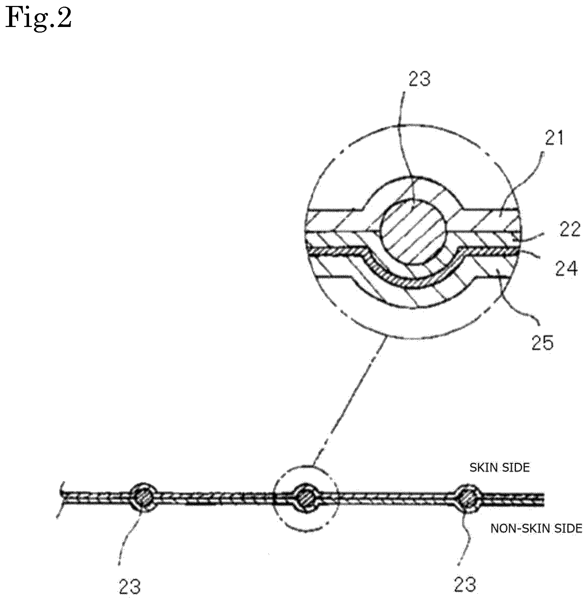

Fig. 1 is a plan view of a stretchable waterproof sheet 20 according to an embodiment of the present invention. Further, Fig. 2 is a cross-sectional view taken along line A-A of Fig. 1. Hereinafter, the stretchable waterproof sheet 20 will be described with reference to Figs. 1 and 2.

The stretchable waterproof sheet 20 is a multi-ply sheet including a first fiber sheet 21, a liquid diffusive sheet 22, an elastic member 23, a moisture-permeable film 24, and a second fiber sheet 25, and these are laminated in a predetermined order. When the stretchable waterproof sheet 20 is applied to a covering article that covers a part of a body, the first fiber sheet 21 is located on a skin side, and the second fiber sheet 25 is located on a non-skin side.

The first fiber sheet 21 is a sheet-shaped nonwoven fabric having air permeability. Examples of the nonwoven fabric include a spunbond nonwoven fabric, a spunlace nonwoven fabric, and a thermal-bond nonwoven fabric. In addition, in a case where the first fiber sheet 21 is required to be hydrophilic, for example, the first fiber sheet 21 may be subjected to a hydrophilization treatment such as adding a hydrophilizing agent. One side of the first fiber sheet 21 corresponds to an outer surface of the stretchable waterproof sheet 20, and the other surface of the first fiber sheet 21 is overlapped and joined to the liquid diffusive sheet 22 via the elastic member 23. Therefore, the first fiber sheet 21 protects the liquid diffusive sheet 22 from physical damage.

The liquid diffusive sheet 22 is a fiber sheet having liquid diffusibility and mainly containing a cellulose-based component. The liquid diffusive sheet 22 is, for example, a paper (a thin paper such as a tissue paper and a crepe paper) obtained through a papermaking process, or an air-laid nonwoven fabric obtained through a fiber-laminating process. As described above, one surface of the liquid diffusive sheet 22 is joined to the first fiber sheet 21 through the elastic member 23, and the other side is joined to the moisture-permeable film 24. The liquid diffusive sheet 22 may be provided with a print layer subjected to printing. Details of the print layer will be described later.

The elastic member 23 applies an elastic force to the first fiber sheet 21, the liquid diffusive sheet 22, the moisture-permeable film 24, and the second fiber sheet 25 in a direction in which they form an uneven surface and contract. The elastic member 23 is linear and cylindrical, and has a circular cross-sectional shape. The elastic member 23 is formed of urethane, silicone, butadiene, or styrene-butadiene-based synthetic rubber, or natural rubber. Further, the elastic member 23 may be a combination of any of these raw materials of the synthetic rubber or the natural rubber. The elastic member 23 is arranged such that a stretched direction is in the same direction as a longitudinal direction of Fig. 1, and the plurality of elastic members 23 are arranged with a predetermined or more interval from each other, in parallel in a short direction orthogonal to the longitudinal direction in the plane of Fig. 1. This causes the elastic members 23 to be arranged in a row.

Meanwhile, the elastic member 23 is not limited to cylindrical, but may have a triangular column shape, a square column shape, other polygonal column shape, or an elliptical column shape. Further, the elastic member 23 may be obtained by bundling or twisting together a plurality of linear members. Furthermore, the elastic member 23 is not limited to linear, but may have a sheet shape.

Further, for the arrangement interval, the plurality of elastic members 23 may be arranged at uniform intervals, or may be arranged at non-uniform intervals. In a case where the arrangement intervals of the plurality of elastic members 23 are non-uniform, the arrangement interval of the elastic members 23 may be narrower as closer to a side edge portion of the stretchable waterproof sheet 20, while the arrangement interval of the elastic members 23 may be wider as closer to a center of the stretchable waterproof sheet 20. Further, conversely, the arrangement interval of the elastic member 23 may be wider as closer to the side edge portion of the stretchable waterproof sheet 20, while the arrangement interval of the elastic members 23 may be narrower as closer to the center of the stretchable waterproof sheet 20.

In addition, a thicknesses of the plurality of elastic members 23 may be all the same, or some or all of the plurality of elastic members 23 may have different thicknesses. Thereby, for example, a contraction force may be strengthened by increasing the thickness of the elastic member 23 as closer to the side edge portion of the stretchable waterproof sheet 20, while the contraction force may be weakened by reducing the thickness of the elastic member 23 as closer to the center of the stretchable waterproof sheet 20. Further, conversely, the contraction force may be weakened by reducing the thickness of the elastic member 23 as closer to the side edge portion of the stretchable waterproof sheet 20, while the contraction force may be strengthened by increasing the thickness of the elastic member 23 as closer to the center of the stretchable waterproof sheet 20.

When being joined to the first fiber sheet 21 and the liquid diffusive sheet 22, the elastic member 23 is stretched and joined at a predetermined magnification. Here, the magnification is defined as a ratio of a length L of the elastic member 23 in a state where the elastic member 23 is applied with an external force and pulled, to a length S of the elastic member 23 in a state where no external force (tensile force) other than gravity is applied to the elastic member 23, that is, L/S. When a stretched state of the elastic member 23 is released, the elastic force of the elastic member 23 causes formation of a non-uniform uneven surface on which recesses and protrusions are alternately repeated, on the stretchable waterproof sheet 20.

The predetermined magnification for stretching the elastic member 23 is preferably 1.2 to 4 times, and more preferably 2 to 3 times. In a case where the magnification is less than 1.2 times, a contraction force is small when the elastic member 23 is released from the stretched state, and the uneven surface cannot be sufficiently formed on the stretchable waterproof sheet 20. Whereas, in a case where the magnification is 4 times or more, a contraction force of the elastic member 23 is too large and the uneven surface formed on the stretchable waterproof sheet 20 becomes too uneven, which also causes poor appearance.

A pitch interval between adjacent protrusions formed on the stretchable waterproof sheet 20 is preferably 2.00 mm to 7.00 mm, and more preferably 3.00 mm to 6.25 mm. By narrowing the pitch interval between adjacent protrusions, a fine uneven surface can be formed, and beautiful appearance can be achieved. Further, the recesses and the protrusions can be formed in a substantially uniform shape, and the shape can be maintained. This eliminates deformation of the uneven surface, and enables enhancement of softness, as well as of moisture transpiration that indicates absorbing and drying of sweat, heat dissipation, and moisture permeability of the stretchable waterproof sheet 20. In addition, a texture becomes satisfactory since a contact area with the skin per unit area decreases, and absorbability of sweat can be enhanced since a surface area per unit volume is increased. Further, since the surface area per unit volume of the stretchable waterproof sheet 20 can be reduced by appropriately suppressing the contraction force of the elastic member 23 and widening the pitch interval between adjacent protrusions, a material cost of the stretchable waterproof sheet 20 can be reduced.

The moisture-permeable film 24 is a microporous film, and has a property of transmitting gas such as vapor, but not transmitting a liquid. One surface of the moisture-permeable film 24 is overlapped and joined to a surface opposite to a surface of the liquid diffusive sheet 22 to which the first fiber sheet 21 is joined. Further, the other surface of the moisture-permeable film 24 is overlapped and joined to a surface of the second fiber sheet 25.

The second fiber sheet 25 is a sheet-shaped nonwoven fabric having air permeability. Examples of the nonwoven fabric include a spunbond nonwoven fabric, a spunlace nonwoven fabric, and a thermal-bond nonwoven fabric. In addition, in a case where the second fiber sheet 25 is required to be water repellent, for example, the second fiber sheet 25 may be subjected to water-repellent treatment such as adding a water repellent agent. One surface of the second fiber sheet 25 is joined to a surface opposite to a surface of the moisture-permeable film 24 to which the liquid diffusive sheet 22 is overlapped and joined. The other surface of the second fiber sheet 25 forms an outer surface opposite to the outer surface of the stretchable waterproof sheet 20 formed by the first fiber sheet 21. Therefore, the second fiber sheet 25 protects the moisture-permeable film 24 from physical damage.

### [About joining method]

Next, a joining method at a time of forming the above-described stretchable waterproof sheet 20 is explained. Examples of the joining method include bonding, heat sealing, and ultrasonic joining, but bonding is preferable from the viewpoint of easy operation. Hereinafter, a case of performing bonding with use of a hot melt adhesive as a joining method will be described. Examples of the hot melt adhesive include adhesives of ethylene-vinyl acetate copolymer (EVA) based, polyolefin (PO) based, polyamide (PA) based, silicone synthetic rubber (SR) based, acrylic (ACR) based, and moisture-curing polyurethane (PUR) based. One of these may be used alone, or two or more may be used in combination. Meanwhile, in addition to the hot melt adhesive, various adhesives such as a pressure-sensitive adhesive, a curable adhesive, an organic solvent based adhesive, and a water-soluble adhesive can also be used.

When the hot melt adhesive is applied in a mist form to a surface of at least one of the liquid diffusive sheet 22 or the moisture-permeable film 24 by spraying, the liquid diffusive sheet 22 and the moisture-permeable film 24 can be partially joined. In addition, when the hot melt adhesive is applied in a mist form to a surface of at least one of the moisture-permeable film 24 or the second fiber sheet 25 by spraying, the moisture-permeable film 24 and the second fiber sheet 25 can be partially joined. By partially applying the hot melt adhesive in a mist form in this way instead of applying on the entire surface, the stretchable waterproof sheet 20 becomes soft, and moisture transpiration, heat dissipation, and moisture permeability of the stretchable waterproof sheet 20 become satisfactory. Note that, examples of the application method of the hot melt adhesive other than spraying in a mist form include a method of applying in a linear shape, a dot shape, a stripe shape, a spiral shape, a block shape, and a pattern shape. Any one method of these may be used, or a plurality of methods may be used in combination.

Between the liquid diffusive sheet 22 and the moisture-permeable film 24 and between the moisture-permeable film 24 and the second fiber sheet 25, a non-adhesive portion having no adhesive layer is formed, and this non-adhesive portion forms a space. This enables enhancement of each performance of moisture transpiration, heat dissipation, and moisture permeability in the stretchable waterproof sheet 20.

Further, the hot melt adhesive is applied to the entire peripheral surface of the elastic member 23 by splaying, the elastic member 23 applied with the hot melt adhesive is arranged between the first fiber sheet 21 and the liquid diffusive sheet 22, and these are laminated and joined. Around the elastic member 23, an adhesive layer is formed. The first fiber sheet 21 and the liquid diffusive sheet 22 are joined by sandwiching the elastic member 23 in between to which the hot melt adhesive is applied. In addition, since the plurality of elastic members 23 are arranged in parallel at predetermined intervals, the first fiber sheet 21 and the liquid diffusive sheet 22 are partially joined.

Note that, although not particularly shown in Fig. 2, on opposing surfaces of the first fiber sheet 21 and the liquid diffusive sheet 22, a region having no elastic member 23 is a non-adhesive portion, and this non-adhesive portion forms a space.

Further, in the above description, a description has been made in which the hot melt adhesive is applied to the entire peripheral surface of the elastic member 23, but the present invention is not limited to this. That is, the hot melt adhesive may be partially applied to at least one of the first fiber sheet 21 or the liquid diffusive sheet 22, and the first fiber sheet 21 and the liquid diffusive sheet 22 may be joined with the elastic member 23 interposed. Also in this case, since the joining between the first fiber sheet 21 and the liquid diffusive sheet 22 is partial, a space is formed between the first fiber sheet 21 and the liquid diffusive sheet 22.

### [About liquid diffusive sheet 22]

For the liquid diffusive sheet 22, pulp paper or a material made of pulp as a main raw material, that is, a material containing a cellulose-based component (hereinafter may be referred to as "paper material") can be used.

As pulp to be a main raw material, any of wood pulp or waste paper pulp, or a combination of at least one or more of them can be used. As wood pulp, for example, bleached softwood kraft pulp obtained from conifers such as red pine, yezo spruce, abies sachalinensis, Douglas fir, hemlock, and spruce can be used. In addition, it is also possible to use pulp obtained by blending bleached hardwood kraft pulp obtained from hardwoods such as beech, oak, birch, eucalyptus, common oak, poplar, and alder, with bleached softwood kraft pulp. However, it is preferable to use bleached softwood kraft pulp alone from the viewpoint of production cost and production efficiency.

Meanwhile, in addition to the paper material described above, for example, natural fibers such as kenaf, bamboo fiber, straw, cotton, cocoon filament, sugar cane, and wool can be used. Moreover, without limiting to these natural fibers including pulp, regenerated fibers such as rayon, viscose rayon, and toilet paper material can also be used. In addition, synthetic fibers such as nylon, polyester (PEs), polyethylene terephthalate (PET), polyethylene (PE), polypropylene (PP) can also be used. One of these may be used alone, or two or more may be used in combination. Further, composite fibers may also be used.

In a case where the liquid diffusive sheet 22 is formed of a material containing pulp as a main raw material, blending of pulp is preferably 30% or more, more preferably 50% or more, and most preferably 80% or more. Setting the blending of the pulp to the above ratio enables enhancement of the softness of the entire stretchable waterproof sheet 20, and enhancement of the production efficiency at the time of production.

Further, increasing the blending ratio of the pulp in the liquid diffusive sheet 22 can reduce a use ratio of a petroleum-based raw material. Therefore, it is possible to reduce an amount of CO₂ generated by incineration and other factors causing a load on the environment when discarding the stretchable waterproof sheet 20. As a result, a load on the environment can be reduced.

Note that the paper material used for the liquid diffusive sheet 22 may be a nonwoven fabric blended with the above-described pulp. For example, a spunlace nonwoven fabric mixed with the above-described pulp can also be used as the paper material.

### [Production method for stretchable waterproof sheet 20]

First, the liquid diffusive sheet 22 wound in a roll shape is unrolled from the roll. The liquid diffusive sheet 22 may be subjected to printing in advance. By applying a mechanical process in which the unrolled liquid diffusive sheet 22 is passed through emboss rolls and pressed between the rolls, a mechanical softening process is performed to make the liquid diffusive sheet 22 soft. This mechanical softening process may be an emboss process using a flat roll, or may be an emboss process using a meshing roll having a large number of protrusions on a roll surface. In the case of the latter emboss process, a large number of fine holes are opened on the liquid diffusive sheet 22. However, the mechanical softening process is performed to such an extent that the liquid diffusive sheet 22 is not broken. In addition, a mechanical process other than the emboss process may be adopted.

Whereas, the moisture-permeable film 24 wound in a roll shape is unrolled from the roll, and the hot melt adhesive is partially sprayed on both sides of the unrolled moisture-permeable film 24. On one side of the moisture-permeable film 24 sprayed with the hot melt adhesive, the liquid diffusive sheet 22 subjected to the mechanical softening process is laminated, and the second fiber sheet 25 unrolled from the roll is laminated on the other surface of the moisture-permeable film 24. They are passed through flat rolls in a state of being laminated to be pressure-bonded, and a joined sheet is produced.

A large number of rows of the elastic member 23 are unrolled from a roll formed by winding a large number of the elastic members 23 in parallel. The elastic member 23 is unrolled in a state of being pulled by a predetermined tensile force. The hot melt adhesive is sprayed on the unrolled elastic member 23. In this case, the adhesive is continuously sprayed over the entire length of the elastic member 23 in a length direction. Further, the adhesive is applied to the entire peripheral surface of the elastic member 23. Whereas, the first fiber sheet 21 wound into a roll shape is unrolled from the roll, this first fiber sheet 21 is fed so as to face the joined sheet, and the elastic member 23 is supplied such that the elastic member 23 applied with the adhesive as described above is sandwiched between the joined sheet and the first fiber sheet 21. In this case, the elastic member 23 is supplied between the surface of the liquid diffusive sheet 22 of the joined sheet and the first fiber sheet 21.

The elastic member 23 is passed through the flat rolls in a state of being sandwiched between the surface of the liquid diffusive sheet 22 of the joined sheet and the first fiber sheet 21. The joined sheet, the elastic member 23, and the first fiber sheet 21 are pressure-bonded by the flat rolls, to be laminated and integrated. Since the first fiber sheet 21 and the liquid diffusive sheet 22 are joined via the elastic member 23, both are partially joined. Thus, between the second fiber sheet 25 and the moisture-permeable film 24, and between the moisture-permeable film 24 and the liquid diffusive sheet 22 are partially joined to each other, and the multi-ply sheet formed by partially joining the first fiber sheet 21 and the liquid diffusive sheet 22 is produced. If necessary, the multi-ply sheet may be passed through emboss rolls to be subjected to the mechanical softening process. By performing this process, the softness of the multi-ply sheet can be further enhanced.

Since the produced multi-ply sheet has a long dimension, cutting is performed to make a length dimension in a longitudinal direction of the multi-ply sheet into a predetermined length. In this cutting, the first fiber sheet 21, the liquid diffusive sheet 22, the elastic member 23, the moisture-permeable film 24, and the second fiber sheet 25 are cut. By cutting the elastic member 23, the elastic member 23 in the pulled state is released from the tensile force and contracts due to a restoring force. Since the multi-ply sheet is subjected to a force in a direction in which the length is shortened due to the contraction stress at this time, an uneven surface is formed on the multi-ply sheet, which causes a shirring part to be formed. Thus, the stretchable waterproof sheet 20 having a large number of shirring parts is produced.

Here, as a description of the configuration of the stretchable waterproof sheet 20, the elastic member 23 is a contraction state due to a restoring force, that is, in an unpulled state. A large number of shirring parts extending in a direction perpendicular to a longitudinal direction of the elastic member 23 in the unpulled state are formed, and a row of shirring parts is formed in pattern on the multi-ply sheet.

By the elastic member 23 disposed inside the multi-ply sheet, elasticity is given to the multi-ply sheet. Therefore, when the stretchable waterproof sheet 20 made of the multi-ply sheet is pulled by a hand in a longitudinal direction in Fig. 1, the stretchable waterproof sheet 20 also extends and expands as the elastic member 23 extends. Further, when the hand is released from this state, the elastic member 23 contracts due to its restoring force, which causes the stretchable waterproof sheet 20 to also return to its original size. As described above, since the stretchable waterproof sheet 20 is stretchable, the stretchable waterproof sheet 20 can excellently fit on the body when being used as a covering article that covers a part of the body.

The stretchable waterproof sheet 20 is suitably used as a raw material for disposable fiber products, and is advantageous as a raw material having unconventional excellent properties. The disposable fiber products to which the stretchable waterproof sheet 20 is applied are not limited to those to be discarded in one use, but also include those to be used repeatedly within a short period depending on applications and purposes. Further, those to be repeatedly used include those that can withstand several washings.

The first fiber sheet 21 and the second fiber sheet 25 both are preferably made of a raw material having air permeability and moisture permeability, and as described above, a nonwoven fabric is preferably used as a fiber material having such air permeability and moisture permeability. This can improve texture.

As the nonwoven fabric above, publicly known nonwoven fabrics generally used conventionally can be used. That is, it is possible to use a publicly known nonwoven fabric having a fiber structure in which fibers are unidirectionally or randomly oriented, and fibers are bound by interlacing, fusion, or adhesion. Examples of the fiber material of the nonwoven fabric include: natural fibers such as plant fibers (cellulose polymers) and animal fibers (protein polymers); refined fibers such as lyocell and tencel; regenerated fibers such as rayon and viscose rayon; semi-synthetic fibers such as acetate; synthetic fibers such as nylon and acrylic fibers; and chemical fibers such as polypropylene (PP), polyethylene (PE), and polyethylene terephthalate (PET). One of these may be used alone, or two or more of these may be used in combination. Further, other materials can be included as needed.

Examples of the raw material of the nonwoven fabric used for the first fiber sheet 21 and the second fiber sheet 25 include, for example, a spunbond nonwoven fabric, a thermal-bond nonwoven fabric, a spunlace nonwoven fabric, a dry nonwoven fabric, a wet nonwoven fabric, a melt-blown nonwoven fabric, a chemical-bonded nonwoven fabric, a needle punch nonwoven fabric, a stitch-bonded nonwoven fabric, and a steam jet nonwoven fabric. Among these, it is preferable to use a spunbond nonwoven fabric, a thermal-bond nonwoven fabric, and a spunlace nonwoven fabric.

A preferable basis weight of the nonwoven fabric to be used for the first fiber sheet 21 and the second fiber sheet 25 is preferably 5 to 60 g/m², and more preferably 7 to 40 g/m². The reason why the basis weight of the nonwoven fabric is preferably in the above range is that, in a case where the basis weight is less than 5 g/m², for example, the hot melt adhesive may exude to the surface of the nonwoven fabric when the hot melt adhesive is applied, while in a case where the basis weight exceeds 60 g/m², a product thickness increases, which deteriorates production efficiency and causes packing work to become troublesome. When the basis weight of the nonwoven fabric is 7 to 40 g/m², a joining strength can be increased by combining a joining method using the hot melt adhesive and a joining method using heat sealing or ultrasonic joining.

As a material of the first fiber sheet 21 and the second fiber sheet 25, materials other than a nonwoven fabric may be used as long as it is made of a raw material having air permeability and moisture permeability. For example, an air-permeable sheet or a moisture-permeable sheet made of synthetic resin can also be used.

The liquid diffusive sheet 22 is preferably imparted with a soft structure by the mechanical softening process. As the mechanical softening process, an emboss process is adopted. As an emboss roll used for the emboss process, a pair of emboss rolls obtained by forming a large number of protrusions on a roll surface are used. When the emboss process is performed using the above-described emboss roll, a large number of fine holes are opened on the liquid diffusive sheet 22. At the same time, the cellulose-based tissue is weakened to give softness to the liquid diffusive sheet 22. The holes may be circular holes, linear holes, or slit holes. The liquid diffusive sheet 22 can be similarly given with softness by providing a rough surface with a large number of protrusions and recesses, without the holes being opened in the liquid diffusive sheet 22.

The emboss roll used for the emboss process is not limited to the roll provided with the protrusions, but a pair of flat rolls having no protrusions and having a smooth roll surface may be used. By passing the liquid diffusive sheet 22 between the flat rolls and pressing the liquid diffusive sheet 22 between the rolls, the cellulose-based tissue is weakened. That is, even without performing a punching process, the cellulose-based tissue is weakened by the pressure between the rolls. The emboss process for imparting the soft structure to the liquid diffusive sheet 22 may also be performed by combining the emboss process with the emboss roll having protrusions on the roll surface and the emboss process with the flat rolls.

As described above, by imparting the soft structure to the liquid diffusive sheet 22, the entire stretchable waterproof sheet 20 also has high softness, that enables satisfactory wearing feeling at a time of wearing a disposable fiber product made of the stretchable waterproof sheet 20. That is, there is no sense of discomfort due to the use of the liquid diffusive sheet 22 as the material of the product, and the user can be satisfied with a soft wearing feeling.

The soft structure imparted to the liquid diffusive sheet 22 produces the following effects. That is, when the liquid diffusive sheet 22 has the soft structure, the entire multi-ply sheet has a soft structure and can be easily deformed. That is, the softness of the entire stretchable waterproof sheet 20 also increases, making it possible to give a softer wear feeling to the user by a covering article such as a supporter made of the stretchable waterproof sheet 20. Further, by giving softness to the paper material, the stretchable waterproof sheet 20 can be given a fluffy feeling.

In addition, the above-described weakening process of the cellulose-based tissue by the above-described emboss process has an effect of imparting further excellent moisture transpiration, heat dissipation, and moisture permeability to the stretchable waterproof sheet 20, as will be described later.

As described above, in a state where the elastic member 23 is sandwiched between the first fiber sheet 21 and the liquid diffusive sheet 22, the hot melt adhesive sprayed and applied to the peripheral surface of each elastic member 23 joins the three members. In this joined state, a part of the hot melt adhesive permeates into the layer of the liquid diffusive sheet 22 to form an adhesive-permeated portion. Since the adhesive-permeated portion is integrated with the hot melt adhesive on the peripheral surface of the elastic member 23, the adhesive-permeated portion exerts an anchor function in the joining of the elastic member 23 and the liquid diffusive sheet 22, which further strengthens the joining between the elastic member 23 and the liquid diffusive sheet 22 by the hot melt adhesive.

Thus, the liquid diffusive sheet 22 is formed with an anchor structure of the adhesive. Therefore, a predetermined joining strength can be obtained even if an amount of the adhesive applied to the elastic member 23 is small, and the overall strength of the stretchable waterproof sheet 20 is enhanced, making it possible to provide the stretchable waterproof sheet 20 with high fastness. Furthermore, since formation of the adhesive-permeated portion by the hot melt adhesive in the layer of the liquid diffusive sheet 22 strengthens the joining force between the liquid diffusive sheet 22 and the elastic member 23, it is possible to prevent an occurrence of so-called "rubber escape" when the elastic member 23 is cut.

### [About print layer]

On the liquid diffusive sheet 22, a print layer is formed as necessary. The print layer is formed on a surface side of the liquid diffusive sheet 22 facing the moisture-permeable film 24. According to the present embodiment, since printing is applied to the liquid diffusive sheet 22 instead of applying printing on the nonwoven fabric, clear pictures can be printed easily and efficiently. This makes it possible to express various designs and messages efficiently and effectively. In particular, by appropriately selecting a design and a pattern to be printed on the liquid diffusive sheet 22, selecting a nonwoven fabric to be used for the second fiber sheet 25, and selecting a forming method of the adhesive layer, it is possible to provide the stretchable waterproof sheet 20 as a whole having high design and aesthetics. Examples of the designs and patterns to be printed on the liquid diffusive sheet 22 include, for example, pictures, colors, patterns, and photographs (hereinafter referred to as "pictures").

On the liquid diffusive sheet 22, pictures are printed by, for example, a method such as inkjet, flexographic printing, or gravure printing. A surface of the print layer may be subjected to an anti-fading treatment, for example, by performing a varnishing process or adding a binder. Examples of the binder include publicly known materials such as polyvinyl alcohol (PVA), carboxymethyl cellulose (CMC), ethylene-vinyl acetate copolymer (EVA), acrylic, and lacquer. In addition, it is also possible to use an ink subjected to an anti-fading treatment.

The print layer is preferably formed by flexographic printing. The flexographic printing method has an advantage that a contact area between a plate and the liquid diffusive sheet 22 can be small, and a printing pressure is low. For this reason, it is easy to peel off the liquid diffusive sheet 22 from the plate, and in particular, it is suitable for forming the print layer when the liquid diffusive sheet 22 is thin. Further, the flexographic printing allows many types of ink to be used, and in this point, there is no disadvantage of being affected by an ink viscosity, as compared to an inkjet printing method in which the ink is ejected from a nozzle. In representing the pictures on the print layer, the pictures printed by the plate are printed as pictures of stretched design in accordance with a state where the multi-ply sheet is stretched in an expansion and contraction direction of the elastic member 23. Then, when the pulled state of the elastic member 23 is released, the pictures of the original intended design (design that is not stretched) are displayed. In the above case, a stretching ratio of the pictures to be printed is determined by an expansion and contraction ratio of the elastic member 23. For example, it is possible to print by stretching a length of the pictures of the original intended design (design that is not stretched) to 1.1 times to 3.5 times.

By applying the emboss process on the liquid diffusive sheet 22 to form a plurality of recesses and protrusions, three-dimensional pictures can be displayed on the print layer, and the pictures of a design different depending on viewing angles can be expressed. In addition, there are a print layer located in a space formed in the non-adhesive portion and a print layer formed at a position covered by the adhesive layer, and the combination of such print layers enables a variety of the design that appears in the appearance. If a ratio of the space is large, the print layer appearing in the appearance will be visually perceived as blurred, and if a ratio of the space is small, the print layer appearing in the appearance is clearly viewed.

Since the print layer is viewed from the outside through the second fiber sheet 25 and the moisture-permeable film 24, various designs and messages can be efficiently and effectively expressed in the stretchable waterproof sheet 20. In order to enable the print layer to be accurately recognized from the outside and to have aesthetics, it is preferable to use a nonwoven fabric as the second fiber sheet 25.

### [Covering article]

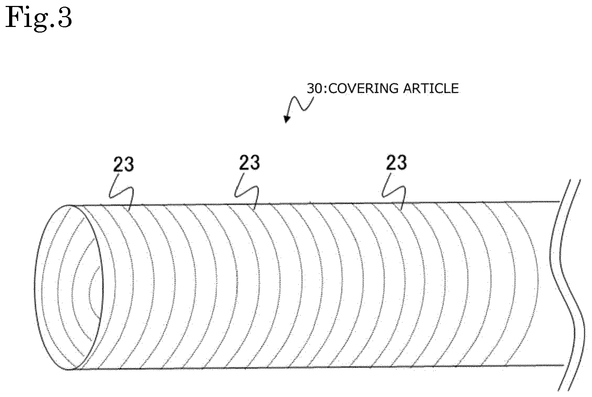

Fig. 3 is a view showing a covering article using the stretchable waterproof sheet 20 shown in Fig. 1. In a covering article 30 shown in Fig. 3, a pair of opposing side edges of the stretchable waterproof sheet 20 are joined to each other, to form a tubular shape with both ends open such that the first fiber sheet 21 of the stretchable waterproof sheet 20 shown in Fig. 1 is to be an inner side, and the elastic member 23 has a substantially annular shape (or helical). At this time, it is not necessary to form the annular shape in a precise manner, and the annular shape may be cut off at a joint portion. For the joining, means such as ultrasonic joining, heat sealing, and joining using an adhesive (for example, a hot melt adhesive) is used. Note that a diameter of the tube (a diameter of the elastic member in the non-extended state) may simply be set to a size according to a wearing area of the user.

As described above, since the covering article 30 can be realized at a low cost with a simple configuration in which the stretchable waterproof sheet 20 is formed into a tubular shape, it can be disposable. Therefore, the covering article 30 may simply be replaced with a new covering article when becoming dirty, making it possible to always keep hygiene.

Moreover, since the covering article 30 includes the moisture-permeable film 24, a wearing area of the covering article 30 can be prevented from getting wet even when water is applied. In addition, since the covering article 30 includes the liquid diffusive sheet 22, the covering article 30 can absorb user's sweat and release vapor to the outside through the moisture-permeable film 24, enabling suppression of sweatiness.

The tubular covering article 30 can be cut into any length and shape by scissors. At this time, no matter how the user cuts the covering article 30, the cut end does not come apart. Therefore, it is possible to freely process in a shape fitting with a wearing area, and the appearance can be made satisfactory since the cut end does not come apart.

Moreover, while the tubular covering article 30 extends in a direction in which the diameter expands, the tubular covering article 30 hardly expands in the longitudinal direction of the tube. Therefore, when being worn on a joint site, the covering article 30 can be used as a supporter because the covering article 30 functions to suppress movement of the joint of the user.

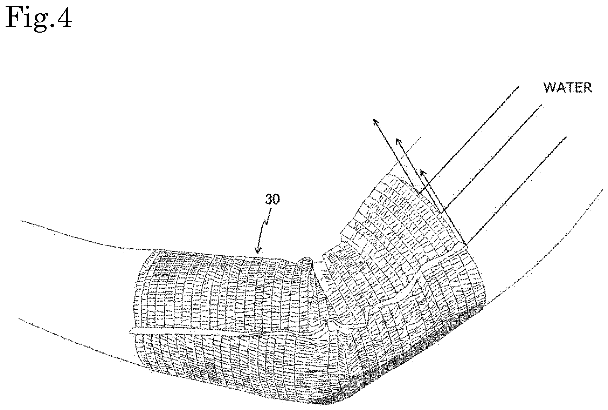

Fig. 4 is a view showing a state where the covering article 30 shown in Fig. 3 is worn on an elbow. As shown in Fig. 4, since each of the annular elastic members 23 arranged substantially in parallel (continuously along the wearing site) in multiple stages press the periphery of the arm, there is close contact between the user's skin and the covering article 30, enabling suppression of water intrusion. That is, even in a case of water intrusion that cannot be prevented only by the elastic member 23 on the opening side of the covering article 30, the water intrusion is blocked in stages, such as the next elastic member 23 and further the next elastic member 23 and so on. In addition, since water does not intrude in this way, a drug does not flow out by the water when the covering article 30 covers the drug. Furthermore, since the plurality of annular elastic members 23 press and fit with the user's skin, the covering article 30 does not easily shift even at moving areas such as joints.

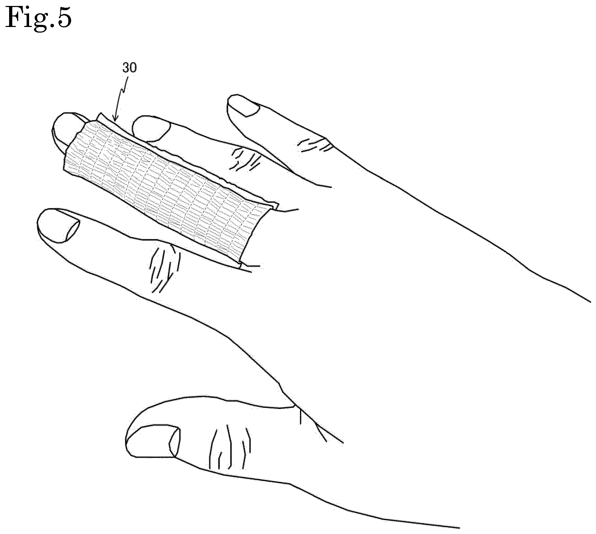

Note that the covering article 30 can fit with various body parts of the user by appropriately setting the size of the diameter. For example, as shown in Fig. 5, the covering article 30 may be worn on a finger of a hand, or worn on an ankle, a calf, a knee, a thigh, a neck, a head, an arm, and a wrist.

### (Other embodiments)

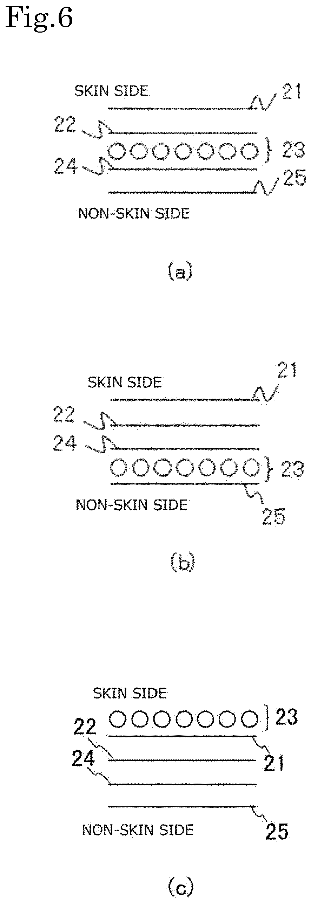

Although a description has been made to the case where the covering article 30 described above has a layer configuration in which the first fiber sheet 21, the elastic member 23, the liquid diffusive sheet 22, the moisture-permeable film 24, and the second fiber sheet 25 are laminated in this order from the skin side, the present invention is not limited to this layer configuration. For example, as shown in Fig. 6(a), a covering article may be such that the first fiber sheet 21, the liquid diffusive sheet 22, the elastic member 23, the moisture-permeable film 24, and the second fiber sheet 25 are laminated in this order from the skin side. Moreover, as shown in Fig. 6(b), a covering article may be such that the first fiber sheet 21, the liquid diffusive sheet 22, the moisture-permeable film 24, the elastic member 23, and the second fiber sheet 25 are laminated in this order from the skin side.

The liquid diffusive sheet 22 is positioned closer to the user's skin than the moisture-permeable film 24 in any of the layer configurations of the covering article described above. This makes it possible to prevent the liquid diffusive sheet from being wetted by water from the outside, and to satisfactorily exhibit moisture transpiration that indicates an index for absorbing and drying user's sweat.

Moreover, in the covering article described above, one or both of the first fiber sheet 21 and the second fiber sheet 25 may be omitted.

Further, although the covering article described above has been explained as a tubular body with both-ends opened, the present invention is not limited to this. For example, the covering article may be a bag body sealed at one end. This can prevent water intrusion more reliably.

Furthermore, as shown in Fig. 6(c), which is an embodiment not according to the invention , the clothing article may be laminated in the order of the elastic member 23, the first fiber sheet 21, the liquid diffusive sheet 22, the moisture-permeable film 24, and the second fiber sheet 25 from the skin side. This causes close contact between the elastic member 23 and the user's skin when the user wears the covering article, enabling effective prevention of water intrusion.

### Reference Signs List

- 20: stretchable waterproof sheet
- 21: first fiber sheet
- 22: liquid diffusive sheet
- 23: elastic member
- 24: moisture-permeable film
- 25: second fiber sheet
- 30: covering article

## Claims

1. A covering article for a body part comprising a stretchable waterproof sheet (20), wherein the stretchable waterproof sheet (20) comprises:
a liquid diffusive sheet (22) having a property of diffusing absorbed liquid, which is a paper material containing a cellulose-based component;
a moisture-permeable film (24) laminated on the liquid diffusive sheet (22); and
a plurality of elastic members (23) joined to at least one of the liquid diffusive sheet (22) or the moisture-permeable film (24) and arranged substantially in parallel, wherein
a pair of opposing side edges of the stretchable waterproof sheet (20) are joined to each other to cause the plurality of elastic members (23) to be substantially annular, to form a tubular shape with both ends open, and
when a user wears, the liquid diffusive sheet (22) is positioned closer to a skin of the user than the moisture-permeable film (24) and the elastic members (23) having a substantially annular shape press a wearing site of the user,
wherein the body part is a finger of a hand, an ankle, a calf, a knee, a thigh, a neck, a head, an arm, or a wrist.

2. The covering article according to claim 1, wherein
the elastic members (23) are positioned closer to a skin of the user than the liquid diffusive sheet (22) when the user wears.

3. The covering article according to claim 1 or 2, wherein
the elastic members (23) have a linear shape, and are joined to at least one of the liquid diffusive sheet (22) or the moisture-permeable film (24) by an adhesive applied on a peripheral surface.

4. The covering article according to claim 3, wherein
a plurality of the elastic members (23) having a substantially annular shape are continuously arranged along a wearing site when the user wears.

5. The covering article according to any one of claims 1 to 4, further comprising:
a fiber sheet (21, 25) laminated on an outer surface of at least one of the liquid diffusive sheet (22) or the moisture-permeable film (24) that are laminated.

6. The covering article according to any one of claims 1 to 5, wherein the paper material comprises 30% or more of pulp.

7. The covering article according to any one of claims 1 to 6, wherein the paper material is a paper.

8. The covering article according to any one of claims 1 to 6, wherein the paper material is a nonwoven fabric blended with pulp.

## Patentansprüche

1. Auflagegegenstand für einen Körperteil, umfassend eine dehnbare wasserfeste Lage (20), wobei die dehnbare wasserfeste Lage (20) umfasst:
eine flüssigkeitsdiffusionsfähige Lage (22) mit einer Eigenschaft zum Diffundieren von absorbierter Flüssigkeit, die ein Papiermaterial ist, das eine Komponente auf Cellulosebasis enthält;
einen feuchtigkeitsdurchlässigen Film (24), der an die flüssigkeitsdiffusionsfähige Lage (22) laminiert ist; und
eine Vielzahl von elastischen Elementen (23), die mit wenigstens einem von der flüssigkeitsdiffusionsfähigen Lage (22) und dem feuchtigkeitsdurchlässigen Film (24) verbunden sind und im Wesentlichen parallel angeordnet sind, wobei
ein Paar von gegenüberliegenden Seitenrändern der dehnbaren wasserfesten Lage (20) miteinander verbunden ist, um zu bewirken, dass die Vielzahl von elastischen Elementen (23) im Wesentlichen ringförmig ist, um eine schlauchförmige Form zu bilden, bei der beide Enden offen sind, und
bei Tragen durch einen Benutzer die flüssigkeitsdiffusionsfähige Lage (22) näher bei der Haut des Benutzers als der feuchtigkeitsdurchlässige Film (24) angeordnet ist und die elastischen Elemente (23), die eine im wesentlichen ringförmige Form aufweisen, eine Tragestelle des Benutzers drücken,
wobei der Körperteil ein Finger einer Hand, ein Knöchel, eine Wade, ein Knie, ein Oberschenkel, ein Hals, ein Kopf, ein Arm oder ein Handgelenk ist.

2. Auflagegegenstand gemäß Anspruch 1, wobei
bei Tragen durch den Benutzer die elastischen Elemente (23) näher bei der Haut des Benutzers als die flüssigkeitsdiffusionsfähige Lage (22) angeordnet sind.

3. Auflagegegenstand gemäß Anspruch 1 oder 2, wobei
die elastischen Elemente (23) eine lineare Form aufweisen und durch einen Klebstoff, der an einer Randoberfläche aufgebracht ist, mit wenigstens einem von der flüssigkeitsdiffusionsfähigen Lage (22) und dem feuchtigkeitsdurchlässigen Film (24) verbunden sind.

4. Auflagegegenstand gemäß Anspruch 3, wobei
bei Tragen durch den Benutzer eine Vielzahl der elastischen Elemente (23), die eine im Wesentlichen ringförmige Form aufweisen, kontinuierlich entlang einer Tragestelle angeordnet sind.

5. Auflagegegenstand gemäß einem der Ansprüche 1 bis 4, ferner umfassend:
eine Faserlage (21, 25), die an eine Außenoberfläche von wenigstens einem von der flüssigkeitsdiffusionsfähigen Lage (22) und dem feuchtigkeitsdurchlässigen Film (24), die laminiert sind, laminiert ist.

6. Auflagegegenstand gemäß einem der Ansprüche 1 bis 5, wobei das Papiermaterial 30 % oder mehr Zellstoff umfasst.

7. Auflagegegenstand gemäß einem der Ansprüche 1 bis 6, wobei das Papiermaterial ein Papier ist.

8. Auflagegegenstand gemäß einem der Ansprüche 1 bis 6, wobei das Papiermaterial ein mit Zellstoff gemischtes Vlies ist.

## Revendications

1. Article de couverture pour une partie de corps comprenant une feuille imperméable à l'eau étirable (20), dans lequel la feuille imperméable à l'eau étirable (20) comprend :
une feuille de diffusion de liquide (22) ayant une propriété de diffusion de liquide absorbé, qui est un matériau en papier contenant un composant à base de cellulose ;
un film perméable à l'humidité (24) stratifié sur la feuille de diffusion de liquide (22) ; et
une pluralité d'éléments élastiques (23) reliés à au moins l'un de la feuille de diffusion de liquide (22) ou du film perméable à l'humidité (24) et agencés sensiblement en parallèle, dans lequel
une paire de bords latéraux opposés de la feuille imperméable à l'eau étirable (20) sont reliés l'un à l'autre pour amener la pluralité d'éléments élastiques (23) à être sensiblement annulaires, pour constituer une forme tubulaire avec les deux extrémités ouvertes, et
lorsqu'un utilisateur porte l'article, la feuille de diffusion de liquide (22) est positionnée plus près de la peau de l'utilisateur que le film perméable à l'humidité (24) et les éléments élastiques (23) ayant une forme sensiblement annulaire pressent un site de port de l'utilisateur,
dans lequel la partie de corps est un doigt d'une main, une cheville, un mollet, un genou, une cuisse, un cou, une tête, un bras ou un poignet.

2. Article de couverture selon la revendication 1, dans lequel
les éléments élastiques (23) sont positionnés plus près de la peau de l'utilisateur que la feuille de diffusion de liquide (22) lorsque l'utilisateur porte l'article.

3. Article de couverture selon la revendication 1 ou 2, dans lequel
les éléments élastiques (23) ont une forme linéaire, et sont reliés à au moins l'un de la feuille de diffusion de liquide (22) ou du film perméable à l'humidité (24) par un adhésif appliqué sur une surface périphérique.

4. Article de couverture selon la revendication 3, dans lequel
une pluralité des éléments élastiques (23) ayant une forme sensiblement annulaire sont agencés en continu le long d'un site de port lorsque l'utilisateur porte l'article.

5. Article de couverture selon l'une quelconque des revendications 1 à 4, comprenant en outre :
une feuille de fibres (21, 25) stratifiée sur une surface externe d'au moins l'un de la feuille de diffusion de liquide (22) ou du film perméable à l'humidité (24) qui sont stratifiés.

6. Article de couverture selon l'une quelconque des revendications 1 à 5, dans lequel le matériau en papier comprend 30% ou plus de pâte.

7. Article de couverture selon l'une quelconque des revendications 1 à 6, dans lequel le matériau en papier est un papier.

8. Article de couverture selon l'une quelconque des revendications 1 à 6, dans lequel le matériau en papier est un tissu non tissé mélangé avec de la pâte.
